# EUROPÄISCHE PATENTSCHRIFT

(11) **EP 2 549 964 B1**
(45) Veröffentlichungstag und Bekanntmachung des Hinweises auf die Patenterteilung: **15.08.2018**
(21) Anmeldenummer: 11712169.9
(22) Anmeldetag: 17.03.2011
(51) Int. Cl.: A61F 9/008

(54) **OPHTHALMOLOGISCHE LASER-BEHANDLUNGSEINRICHTUNG**
OPHTHALMOLOGICAL LASER TREATMENT DEVICE
DISPOSITIF OPHTALMOLOGIQUE DE TRAITEMENT PAR LASER

(30) Priorität: 20.03.2010 DE 102010012616
(43) Veröffentlichungstag der Anmeldung: 30.01.2013
(73) Patentinhaber: Carl Zeiss Meditec AG, 07745 Jena (DE)
(72) Erfinder: BISCHOFF, Mark, 07749 Jena (DE); STOBRAWA, Gregor, 07743 Jena (DE)
(74) Vertreter: Loritz, Rainer
(86) Internationale Anmeldenummer: PCT/EP2011/001319
(87) Internationale Veröffentlichungsnummer: WO 2011/116900

(56) Entgegenhaltungen:
- WO-A1-99/44492
- WO-A1-2008/055604
- US-A- 5 620 436
- US-A1- 2002 049 431
- US-A1- 2003 120 266
- US-A1- 2003 163 122
- US-B1- 6 267 756

## Beschreibung

Die Erfindung betrifft eine ophthalmologische Laser-Behandlungseinrichtung mit einem Behandlungslaser, insbesondere einem Excimerlaser oder einem Femtosekunden-Laser, zum Eintragen von Energie in einen Teil eines Auges eines Patienten gemäß einer vorgegebenen, im Auge zu erzeugenden chirurgischen Struktur, einer Lichtquelle zum Beleuchten zumindest des Teils des Auges und einer Detektionseinrichtung zum Aufnehmen eines Bildes zumindest des Teils des Auges sowie ein Verfahren zum Eintragen von Energie in ein Auge gemäß einer vorgegebenen chirurgischen Struktur mittels eines Excimerlasers oder eines Femtosekunden-Lasers, insbesondere Betriebsverfahren für eine ophthalmologische Laser-Behandlungseinrichtung, die einen Behandlungslaser und eine Detektionseinrichtung zum Aufnehmen eines Bildes des Auges aufweist

Die chirurgische Struktur kann beispielsweise in Form von Bestrahlungssteuerdatensätzen wie Schussposition, Schussintensität und Schussfrequenz vorgegeben sein. Alternativ kann es sich um abstraktere Angaben wie parametrisierte Raumkurven handeln, die als Vorstufe für Bestrahlungssteuerdaten die zu erzeugenden Schnitte beschreiben. Auch jede andere Form der Darstellung der im Auge zu erzeugenden chirurgischen Struktur ist geeignet. Als Detektionseinrichtung kann beispielsweise eine Digitalkamera verwendet werden.

Mittels ophthalmologischer Laser-Behandlungseinrichtungen können in der Cornea laserchirurgische Verfahren wie die Femtosekunden-Lentikel-Extraktion (engl. "Femto-second Lenticle Extraction"; FLEx), insbesondere mit kleinen Schnitten (engl. "Small Incision Femto-second Lenticle Extraction"; SMILE), durchgeführt werden. Ein entsprechendes Femtosekunden-Lasersystem ist in WO 2008/064771 A1 beschrieben. Hier wird der für eine refraktive Korrektur erforderliche Abtrag stromalen Gewebes durch eine zweifache Laser-Schnittführung zur Präparation eines Lentikels separiert. Die Zone der eigentlichen refraktiven Korrektur (nachfolgend als Korrekturzone bezeichnet) ist dabei vollständig im Lentikel enthalten, das Lentikel kann aber größer sein als die Korrekturzone. Die Größe Korrekturzone und die Größe des Lentikels werden in Abhängigkeit der optischen und physiologischen Gegebenheiten so gewählt, dass die refraktive Korrektur innerhalb der Korrekturzone unabhängig von der Situation im Übergang vom Rand der Korrekturzone zum Rand des Lentikels ist. Beispielsweise kann hier eine Formanpassung notwendig sein, so dass kein Beitrag zur refraktiven Korrektur möglich ist.

Das Lentikel kann dann mit Hilfe einer Pinzette nach dem Öffnen eines Hornhautlappens (engl "flap"), der das Schnittgebiet überdeckt, oder alternativ durch einen kleinen seitlichen Laser-Schnitt hindurch entnommen werden. Dadurch ist nur ein fs-Lasersystem erforderlich. Alternativ ist es beispielsweise aus US 2006/0155265 A1 bekannt, unter einem mittels eines fs-Lasersystems geschnittenen und dann aufgeklappten Hornhautlappen mittels eines zusätzlichen Excimerlasers refraktive Korrekturen in der Cornea vorzunehmen. Anschließend wird der Hornhautlappen wieder zugeklappt.

Aus WO 2006/051364 A1 ist eine weitere Möglichkeit bekannt, um Fehlsichtigkeit laserchirurgisch zu verringern. In diesem Verfahren werden mit einem Femtosekunden-Laser tiefe Schnitte in stromalem Gewebe vorgenommen, um ohne Ablation von Gewebe einen zusammenhängenden Hohlraum zu schaffen, insbesondere mit zylindrischer Form. Beim Zusammenbrechen des Hohlraums kommt es durch die Verminderung der Gewebefestigkeit und den intraokularen Druck zur Entspannung der Cornea und diese nimmt eine neue Form mit veränderter Krümmung an.

Es besteht generell die Notwendigkeit, die mittels Excimer- oder Femtosekunden-Laser in das Auge einzutragende Struktur im Koordinatensystem des Lasers exakt zu positionieren. Die erforderliche Genauigkeit ist dabei von der Art der Behandlung abhängig. Erfolgt keine Korrektur von Aberrationen höherer Ordnung als Sphäre und Zylinder, so ist die Genauigkeitsanforderung bei geschickter Gestaltung der chirurgischen Struktur (auch als "Profil" bezeichnet) gering, also etwa 0.2 mm.

In WO 2008/055604 A1 wird beschrieben, wie die Position des zu behandelnden Auges relativ zum Behandlungslaser durch Verschiebung einer Lagerungseinrichtung für den Patienten durch Aufnahme von Überwachungsbildern des Auges und Erkennung der Pupille erfolgen kann. Die Ist-Lage der Pupille wird dazu mit einer vorgegebenen Soll-Lage verglichen und eine Verschiebung ermittelt, beispielsweise zwischen dem Ist-Mittelpunkt und dem Soll-Mittelpunkt der Pupille. Diese Verschiebung kann entweder automatisch durch Bewegung der Lagerungseinrichtung kompensiert werden oder es können dem Bediener Hinweise zu einer manuellen Kompensationsbewegung gegeben werden.

Trotz der Verschiebungskompensation kann es zu einer suboptimalen Behandlung kommen, nach der das Sehvermögen nicht optimal ist, insbesondere in dunklen Umgebungen.

Der Erfindung liegt die Aufgabe zugrunde, ein ophthalmologische Laser-Behandlungseinrichtung und ein Verfahren der eingangs genannten Art zu verbessern, so dass das Sehvermögen nach einer laserchirurgischen Behandlung verbessert wird.

Die Aufgabe wird gelöst durch eine ophthalmologische Laser-Behandlungseinrichtung, welche die in Anspruch 1 angegebenen Merkmale aufweist, und durch ein Verfahren, welches die in Anspruch 8 angegebenen Merkmale aufweist.

Vorteilhafte Ausgestaltungen der Erfindung sind in den Unteransprüchen angegeben.

Erfindungsgemäß ist eine Auswerteeinheit vorgesehen, die eingerichtet ist zum Ermitteln eines Grades einer momentanen Überlappung einer optischen Zone des Auges und der Struktur oder zumindest eines refraktiv korrigierend wirkenden Teils der Struktur anhand eines aufgenommenen Bildes. Die optische Zone ist im Fall der Behandlung der Cornea die Projektion der (insbesondere maximal geweiteten) Pupillenöffnung auf die Cornea beziehungsweise im allgemeinen Fall generell diejenige Region des zu behandelnden Gewebes, durch die über die (insbesondere maximal geweitete) Pupillenöffnung Licht ins Auge gelangen und zur Bildgebung beitragen kann. Die momentane Überlappung hängt also von der momentanen Öffnungsweite der Pupille ab. Erfindungsgemäß kann darüber hinaus der ermittelte Grad bei mindestens einem der Schritte Ermitteln von Bestrahlungssteuerdaten und Entscheidung über einen Beginn einer Bestrahlung des Auges berücksichtigt werden. Der Grad der Überlappung kann beispielsweise als ein- oder mehrdimensionaler Wert (Skalar, Vektor oder Tensor höherer Stufe) ermittelt werden.

Erfindungsgemäß wurde erkannt, dass es unabhängig von der Art der Positionierung (voll- oder halbautomatisch oder manuell) durch die Ermittlung des Grads der Überlappung zwischen (momentaner) optischer Zone und einzutragender Struktur oder zumindest des refraktiv korrigierend wirkenden Teils der Struktur gelingt, die Überdeckung der optischen Zone durch das mittels Laserbearbeitung gezielt veränderte Gewebevolumen zu kontrollieren und so eine vollständige Überdeckung zu ermöglichen, was für eine erfolgreiche refraktive Behandlung ohne Beeinträchtigung des Sehvermögens wichtig ist. Erfolgt - wie im Stand der Technik - keine Kontrolle der Überlappung, so kann bei geringer Umgebungshelligkeit das Sehvermögen beeinträchtigt sein. Erfolgt der Vergleich der Überlappung von Pupillen und Behandlungsgeometrie automatisch, so kann sich der Anwender vollständig auf die Zentrieraufgabe konzentrieren.

Bei der einzutragenden Struktur kann es sich beispielsweise um ein im Anschluss an die Laserbearbeitung manuell zu entfernendes Gewebevolumen (Lentikel) handeln. Es kann sich beispielsweise auch um ein Schnittmuster für die radiale Keratektomie handeln oder um zylindrische oder kegelförmige Schnitte. Meist haben diese Strukturen ein Symmetriezentrum, das etwa im Mittelpunkt der optischen Zone positioniert werden sollte. Solche einzutragenden Strukturen weisen im Zentrum der Struktur oft gerade keine Schnitte auf.

Vorzugsweise ermittelt die Auswerteeinheit zum Ermitteln des Grades der Überlappung eine Fläche der Pupille anhand des aufgenommenen Bildes und eine Fläche der Struktur oder zumindest des refraktiv korrigierend wirkenden Teils der Struktur und eine Schnittmenge der beiden Flächen. Zweckmäßigerweise werden zum Ermitteln der Pupillenfläche Form und Lage der Pupille ermittelt. Die Flächen können beispielsweise als Skalare (Betrag der betreffenden Fläche), als ebene Umrisskurven, als parametrisierte räumliche Vektorflächen oder als Punktwolke diskreter Stützstellen ermittelt werden. Anhand der Schnittmenge kann mit geringem Aufwand der Überlappungsgrad ermittelt werden, beispielsweise durch Ermittlung des Quotienten aus der Flächengröße einer ebenen Projektion der Schnittmenge und der Flächengröße der Pupille. Bei der Bestimmung der Flächen und der Schnittmenge ist notwendigerweise der Abbildungsmaßstab des aufgenommenen Bildes zu berücksichtigen.

Vorteilhafterweise ist die Auswerteeinheit eingerichtet zum Identifizieren und Lokalisieren eines Charakteristikums des Auges in dem aufgenommenen Bild und zum Ermitteln einer relativen Verschiebung zwischen einem Punkt des Charakteristikums und einem Punkt der Struktur. Als Charakteristikum kann beispielsweise die Pupille, der Pupillenrand, der Flächenschwerpunkt der Pupille, ein bestangepasster Kreis oder eine bestangepasste Ellipse identifiziert werden. Insbesondere kann eine visuelle und/oder akustische Ausgabe der Verschiebung oder einer entgegengerichtet gleichgroßen Verschiebung und/oder des Grads der Überlappung erfolgen. Die Verschiebung kann vorteilhafterweise bei der Bewertung der Überlappungssituation verwendet werden.

Um eine ausreichende Überdeckung der optischen Zone durch die chirurgische Struktur (nachfolgend auch als Bearbeitungsgebiet bezeichnet) zu ermöglichen kann die einzutragende Struktur, beispielsweise Randschnitte eines Lentikels, verschoben und/oder vergrößert werden, insbesondere bis eine vollständige Überlappung mit der Pupille prognostiziert wird. Dies kann manuell, halb- oder auch vollautomatisch (durch die Auswerteeinheit oder eine Steuereinheit) erfolgen. Die Vergrößerung des Bearbeitungsgebietes (im Beispiel: die eigentliche Korrekturzone innerhalb des Lentikeldurchmessers) gegenüber der optischen Zone (dem insbesondere maximalen Pupillendurchmesser) um einen Betrag, welcher mindestens der Verschiebung des Bearbeitungszentrums (Lentikelzentrums) gegenüber dem Zentrum der optischen Zone (skotopisches Pupillenzentrum) entspricht, was als Dezentrierung bezeichnet werden kann.

Besonders vorteilhaft ist es daher, wenn die Auswerteeinheit einen Grad der Eignung der momentanen Überdeckungssituation zwischen Struktur und optischer Zone anhand des ermittelten Überlappungsgrades bestimmt und ausgibt. Das erleichtert dem Behandler die Bewertung der Überdeckungssituation und kann so die Behandlungsdauer verkürzen, was die Wahrscheinlichkeit für zwischenzeitige Lageveränderungen durch den Patienten minimiert.

Bevorzugt sind Ausführungsformen, in denen die Auswerteeinheit das aufgenommene Bild in Überlagerung mit einer grafischen Darstellung der Struktur gemäß der ermittelten Verschiebung visuell ausgibt. Dadurch kann der Behandler den Grad der Überlagerung und insbesondere die Überdeckungssituation insgesamt besser und schneller interpretieren, was die Behandlungsdauer weiter verkürzt.

Zweckmäßigerweise ist eine Lagerungseinrichtung für den Patienten und eine Positioniereinrichtung zum Verschieben der Lagerungseinrichtung und/oder des Lasers vorgesehen, wobei die Auswerteeinheit die Positioniereinrichtung in Abhängigkeit des ermittelten Grades der Überlappung von Pupille und Struktur und/oder der Verschiebung zwischen Charakteristikum und Struktur steuert. Dadurch kann der Ort, an dem die Struktur erzeugt wird, relativ zum Auge verschoben werden, ohne die zu erzeugende Struktur selbst in sich zu verändern. Im Koordinatensystem des Lasers erfolgt lediglich eine Translation der Struktur. Das ermöglicht die halb- oder vollautomatische Kompensation einer mangelnden Überlappung und kann ebenfalls der Verkürzung der Behandlungsdauer dienen.

Vorteilhaft sind Ausgestaltungen, in denen die Auswerteeinheit einen Wert des ermittelten Grades mit einem vorgegebenen Schwellwert vergleicht und in Abhängigkeit eines Ergebnisses des Vergleichs ein haptisches und/oder visuelles und/oder akustisches Signal ausgibt. So kann beispielsweise der Behandler über eine akzeptable Überdeckung informiert oder vor einer zu geringen Überdeckung gewarnt werden. Alternativ oder zusätzlich kann die Auswerteeinheit einen Wert des ermittelten Grades mit einem vorgegebenen Schwellwert vergleichen und in Abhängigkeit eines Ergebnisses des Vergleichs, insbesondere auch in Abhängigkeit einer relativen Verschiebung zwischen einem Charakteristikum und der Struktur, eine Bestrahlung des Auges gemäß der Struktur durchführt, insbesondere mit Identität der beiden Schwellwerte. Ein solcher Vergleich kann zweckmäßigerweise stattfinden, nachdem eine Verschiebung zwischen Laser und Auge zur Kompensation einer mangelnden Überdeckung durchgeführt wurde. Dies ermöglicht - sofern die Bedingungen für Verschiebung und Überlappung erfüllt sind - insbesondere eine vollautomatische Behandlung, so dass die Wahrscheinlichkeit für zwischenzeitige Lageveränderungen durch den Patienten minimiert wird.

Vorzugsweise wird das Auge mit Infrarotlicht beleuchtet. Dadurch nimmt die Pupille ihre maximale Öffnungsweite ein, so dass die momentane optische Zone ihre Maximalgröße erreicht. Alternativ kann das Auge zur Aufnahme des Bildes mit sichtbarem Licht beleuchtet werden, wobei eine Intensität des Lichts ermittelt und anhand einer momentanen Fläche des aufgenommenen Bildes und der Intensität eine maximale Pupillenfläche prognostiziert und zum Ermitteln der Überlappung verwendet wird. Die maximale Pupillenweitung kann bei Beleuchtung mit sichtbarem Licht alternativ auch medikamentös erreicht werden, bedeutet aber nach der Behandlung eine vorübergehende Belastung für den Patienten.

In bevorzugten Ausführungsformen umfasst die ophthalmologische Behandlungseinrichtung ein Kontaktelement zur mechanischen Fixierung des Auges. Das Kontaktelement, typischerweise ein Kontaktglas, ist zweckmäßigerweise transparent für den bei der therapeutischen Bestrahlung verwendeten Spektralbereich. Indem das Auge mechanisch fixiert wird, kann die Gefahr einer falschen Positionierung der chirurgischen Struktur im Auge verringert werden. Die mechanische Fixierung ist insbesondere bei Femtosekunden-Lasern erforderlich.

Vorteilhafterweise kann das Ermitteln des Grades der momentanen Überlappung vor und/oder nach dem mechanischen Fixieren durchgeführt werden. Die Ermittlung des Überlappungsgrades vor dem Fixieren kann zur Ermittlung einer Verschiebung des Patienten, die notwendig ist, um einen vorgegeben Grad einer Überlappung zu erreichen, dienen. Zu diesem Zweck kann beispielsweise durch eine rechnerische Simulation anhand eines mathematischen Modells ein Grad einer Überlappung in Abhängigkeit einer Verschiebung prognostiziert werden. Anschließend kann beispielsweise diejenige Verschiebung ermittelt werden, für die ein Maximum des Überlappungsgrades prognostiziert wird. Die Verschiebung des Patienten mit anschließender Fixierung des Auges kann dann beispielsweise automatisch durchgeführt werden oder erst nach einer Bestätigung durch den Behandler. Alternativ kann die Verschiebung des Patienten dem Behandler lediglich als Vorschlag ausgegeben werden. Der Behandler muss dann die Fixierung manuell auslösen.

Der Limbus corneae ist als eindeutige Bewegungsverfolgungsreferenz des Auges bezüglich der Hornhaut (Cornea) geeignet. Ist er nicht sichtbar, bleibt nur die Pupille als Hauptgeometriemerkmal. Die Größenveränderlichkeit der Pupille stellt hierbei jedoch einen Nachteil dar. Mit der Größenänderung verschiebt sich auch das Zentrum der Pupille (engl. "pupil center shift"). Daher ist es von Vorteil, während des Andockvorgangs an ein Kontaktelement und/oder danach Bewegungen des Limbus zu verfolgen, da dieser eine feste Beziehung zur Augengeometrie hat. Kennt man insbesondere die Lage des photopischen und des scotopischen Pupillenzentrums in Bezug auf den Limbus, so kann für jeden beliebigen Pupillendurchmesser durch Interpolation die Lage der scotopischen Pupille ermittelt und die Überlappung danach bewertet werden.

Die Erfindung umfasst auch eine Steuereinheit, die zur Durchführung eines erfindungsgemäßen Verfahrens eingerichtet ist. Die Einrichtung kann programmtechnisch vorliegen, beispielsweise durch Softwaremodule zum Ermitteln eines Grades einer momentanen Überlappung einer optischen Zone des Auges und der Struktur anhand des aufgenommenen Bildes und zum Ermitteln von Bestrahlungssteuerdaten und/oder zur Entscheidung über einen Beginn einer Bestrahlung des Auges in Abhängigkeit des ermittelten Grades.

Die Erfindung ist nicht nur zur Anwendung an der Cornea vorgesehen, sondern kann an allen Teilen des Vorderauges angewendet werden, beispielsweise an der Augenlinse oder dem Kapselsack.

Nachfolgend wird die Erfindung anhand von Ausführungsbeispielen näher erläutert.

In den Zeichnungen zeigen:
Fig. 1 eine ophthalmologische Laser-Behandlungseinrichtung,
Fig. 2 ein Flussdiagramm eines Verfahrens zur Steuerung einer Kompensationsbewegung und
Fig. 3 Varianten der Zentrierung eines Lentikels.

In allen Zeichnungen tragen übereinstimmende Teile gleiche Bezugszeichen.

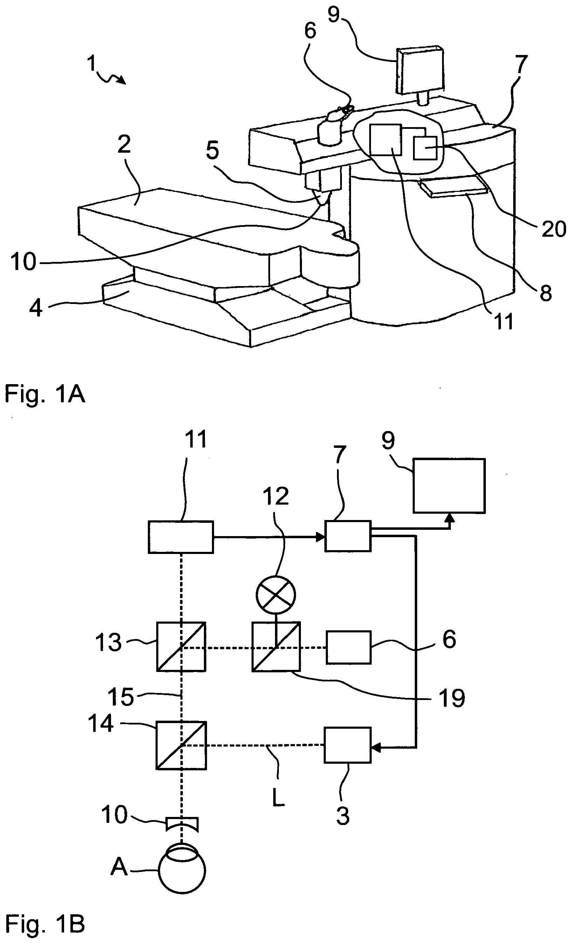

**Fig. 1** zeigt eine ophthalmologische Laser-Behandlungseinrichtung 1 zur Fehlsichtigkeitskorrektur unter Verwendung von Laserstrahlung. In Teilfigur 1A ist das äußere Erscheinungsbild schematisch dargestellt. Die Behandlungseinrichtung 1 umfasst eine Lagerungseinrichtung 2 für einen Patienten in Form einer Liege und einen Behandlungslaser 3 mit Optiken zum Positionieren und Fokussieren des Laserstrahls im Bereich einer Behandlungsposition, an der ein Auge des Patienten angeordnet werden kann. Sie umfasst weiterhin eine Positioniereinrichtung 4, die die Liege 2 trägt und sie in allen drei Raumrichtungen linear bewegen kann. In den Strahlengang des Behandlungslasers 3, der am Behandlungskopf 5, der über der Liege 2 angeordnet ist, austritt, ist ein Mikroskop-Strahlengang eingekoppelt, der dem Behandler an einem Beobachtungsokular 6 die visuelle Kontrolle des Behandlungsfortschritts erlaubt.

Ferner umfasst die Behandlungseinrichtung 1 einen Rechner (engl. "computer") als Steuereinheit 7 mit einer Tastatur 8 und einem Monitor als Anzeige 9. Die ophthalmologische Einrichtung 1 wird mittels der Steuereinheit 7 gesteuert. Der Laser 3 ist beispielsweise ein Femtosekunden-Laser, so dass sowohl ein Flap oder ein seitlicher Entnahmeschnitt als auch ein Lentikel mit demselben Laser geschnitten werden können. Der Behandlungskopf 5 weist an seinem der Liege 2 zugewandten Ende ein Kontaktelement 10 in Form eines Kontaktglases auf, das während der Behandlung das Auge des Patienten berührt und relativ zur Behandlungseinrichtung 1 räumlich fixiert. Während des Bestrahlungsvorgangs wird die Laserstrahlung durch das Kontaktelement 10 hindurch in das Auge des Patienten fokussiert. Das Kontaktelement 10 kann augenseitig beispielsweise plan oder anatomisch gekrümmt sein.

In alternativen Ausführungsformen kann anstelle des oder zusätzlich zu dem Femtosekunden-Lasers ein Excimerlaser angeordnet sein. Sofern ausschließlich ein Excimerlaser verwendet wird, kann auf ein Kontaktelement 10 verzichtet werden. Beim Einsatz eines Excimerlasers ist ein Bewegungsverfolgungssystem zweckmäßig, das den Behandlungslaser 3 während des Bestrahlungsvorgangs eventuellen Augenbewegungen des Patienten nachführt.

In Teilfig. 1B sind schematisch und hinsichtlich des optischen Aufbaus stark vereinfacht die gekoppelten Strahlengänge des Behandlungslasers 3, des Mikroskops 6, der Detektionseinrichtung 11 sowie der Lichtquelle 12 dargestellt. Die Detektionseinrichtung 11 ist als Kamera ausgebildet, mit der das Auge A des Patienten durch zwei Strahlteiler 13, 14 und das Kontaktglas 10, das hier vom Auge A noch beabstandet ist, aufgenommen wird. Es verläuft somit ein Beobachtungsstrahlengang 15 vom Auge A durch das Kontaktglas 10 und beide Strahlteiler 13 und 14 bis zur Kamera 11. Das mittels der Kamera 11 aufgenommene Bild wird zum Computer 7 übertragen. Der Strahlteiler 13 dient dazu, über das Beobachtungsokular 6 das Auge A mikroskopisch beobachten zu können. Über den Strahlteiler 14 kann Behandlungs-Laserstrahlung L vom Laser 3 zum Auge A geleitet werden, wenn dieses mittels des Kontaktglases 10 räumlich fixiert ist, um die hier gewünschte Fehlsichtigkeitskorrektur durchzuführen.

Die Lichtquelle 12 emittiert vorzugsweise ausschließlich Infrarotstrahlung, da sich in diesem Fall die Pupille weit öffnen kann und zudem eine kontraststarke Videoaufnahme der Pupille, insbesondere auch bei sehr dunkler Iris, erfolgen kann. Die Lichtquelle 12 kann aber auch zusätzlich oder alternativ sichtbares Licht emittieren. Insbesondere kann der sichtbare Spektralanteil mittels eines Filters (nicht abgebildet) abschaltbar sein. Das Licht der Lichtquelle 12 wird über einen weiteren Strahlteiler 19 beispielsweise in den Mikroskopstrahlengang eingespiegelt.

Wenn das Auge des Patienten durch die Einrichtung 7 erfasst wird, vorzugsweise während des Andockvorgangs an das Kontaktelement 10 oder unmittelbar danach, wird durch die Auswerteeinheit 20 das Videobild der Kamera 11 analysiert. Zu diesem Zweck führt sie eine Pupillenerkennung durch und zeigt zumindest ein geometrisches Charakteristikum, beispielsweise Pupillenrand, Flächenschwerpunkt, beste Kreisanpassung (engl. "fit") oder beste Ellipsenanpassung, auf dem Monitor 9 und/oder im Okular 6 dem Bediener in Überlagerung mit dem Videobild an. Zusätzlich oder alternativ vergleicht sie ein geometrisches Charakteristikum mit mindestens einem Behandlungsparameter, beispielsweise der Lentikelposition, der Lage und Form der Korrekturzone des Lentikels, dem Lentikeldurchmesser, dem Randschnittwinkel, dem Flapdurchmesser, der Flapmitte, der Position des Flapscharniers (engl. "hinge") oder dem Winkel des Scharniers, und/oder einem Systemparameter, beispielsweise dem Mittelpunkt des Behandlungsgebiets, und bestimmt eine Abweichung von einem vorgegebenen Idealfall und gibt sie auf dem Monitor 9 aus.

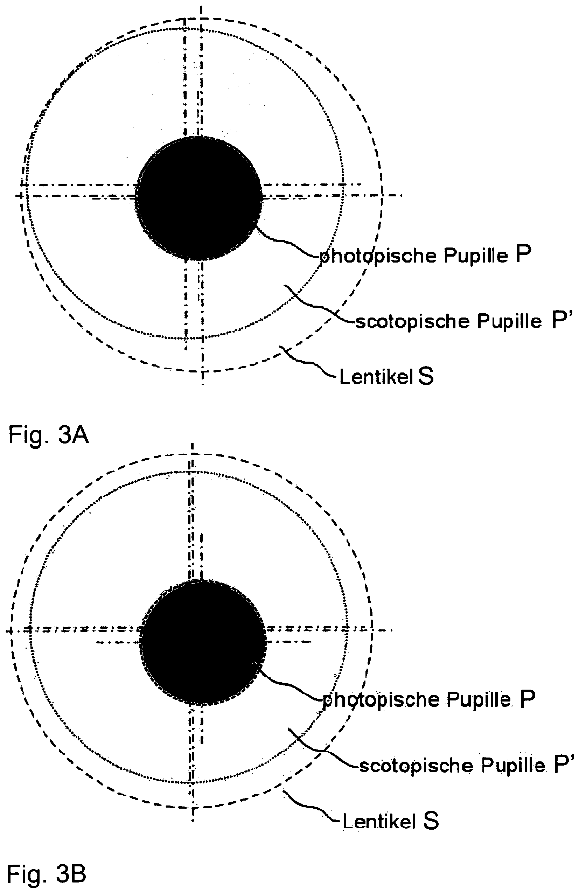

Beispielsweise werden bei der Analyse mittels Bestimmung des Flächenverhältnisses von Pupille und entsprechend skalierter chirurgischer Struktur ein Grad der Überlappung zwischen zu erzeugender Struktur oder zumindest dem refraktiv korrigierend wirkenden Teil der Struktur und optischer Zone sowie eine Verschiebung zwischen dem Mittelpunkt der Pupille als beispielhaftem Referenzpunkt und dem Mittelpunkt der zu erzeugenden Struktur ermittelt. Diese kann dazu verwendet werden, die Positioniereinrichtung 4 so anzusteuern, dass die Liege 2 und somit das Auge A des auf der Liege 2 liegenden Patienten in eine vorbestimmte Soll-Position relativ zum Kontaktglas 10 gebracht werden kann. Die Verschiebung kann anstelle der Pupillenmitte alternativ mit jedem anderen augenfesten Charakteristikum ermittelt werden, indem diese in dem aufgenommenen Bild identifiziert und lokalisiert wird. Zur Beschreibung der Ermittlung der Verschiebung wird auf WO 2008/055604 A1 verwiesen, dort insbesondere auf Fig. 3 und die zugehörigen Erläuterungen. Zweckmäßigerweise erfolgen Bildaufnahme und Identifikation sowie Lokalisierung des Charakteristikums wiederholt, um Lageänderungen des Auges berücksichtigen zu können. Dies gilt auch für die Ermittlung des momentanen Grades der Überlappung.

Die Flächen können beispielsweise durch Zählen von Bildelementen (engl. "picture elements", pixels) in digitalisierten Bildern ermittelt werden, beispielsweise der Schnittmengenpixel, die sowohl innerhalb ermittelten Pupillenrandkurve als auch innerhalb der Korrekturzone der chirurgischen Struktur liegt. Das Verhältnis der Flächeninhalte der gesamten Pupillenfläche mit dem Anteil der Pupillenfläche, welcher mit der Korrekturzone zusammenfällt, ergibt den Überlappungsgrad. Dieser beträgt gerade dann 100%, wenn die Pupille sich vollständig in der Korrekturzone befindet. Zum Grad der Eignung kann weiterhin die Abweichung der Schwerpunkte von Korrekturzone und Pupillenfläche herangezogen werden, welche eine Aussage über die Überlappungsreserve (zusätzliche Überdeckung der Korrekturzone über die Pupillenfläche hinaus) erlaubt. Es ist aber auch denkbar, bei bereits partieller Überlappung von beispielsweise nur 90% oder 95% ein Signal für eine ausreichende Eignung auszugeben.

Die Genauigkeit des Vergleichs zwischen Behandlungsparametern und charakteristischen geometrischen Größen des Videobildes kann durch eine zusätzliche Anpassung der Skalierung des Videobildes und/oder der überlagerten visualisierten Behandlungsparameter weiter verbessert werden. Die Skalierung sorgt dabei für eine bessere Übereinstimmung geometrische Übereinstimmung der Metrik der zu vergleichenden Koordinatensysteme. Zudem kann eine Bewertung der Abweichung erfolgen, insbesondere unter dem Gesichtspunkt, ob die aktuelle Position einen vorgegebenen Schwellwert für die Dezentrierung nicht überschreitet und/oder einen vorgegebenen Schwellwert für die Überlappung der optischen Zone mit der zu erzeugenden Struktur nicht unterschreitet.

Die Auswerteeinheit 20, die beispielsweise ein Softwaremodul der Steuereinheit 7 ist, zeigt beispielsweise das jeweils zuletzt aufgenommene Bild unter Markierung des erkannten Charakteristikums, hier des Pupillenrandes, mit Überlagerung der zu erzeugenden Struktur auf dem Monitor 9 an. Wahlweise kann die zu erzeugende Struktur und die Markierung des erkannten Charakteristikums auch in den Mikroskopstrahlengang eingespiegelt werden, so dass der Bediener diese Entscheidungshilfen direkt mit dem Bild des Auges A überlagert wahrnimmt. Er kann dadurch den Grad der Überlappung unmittelbar selbst wahrnehmen.

In einer speziellen Ausführung kann basierend auf der Bewertung kann eine automatische Reaktion des Gerätes eingeleitet werden, beispielsweise eine Kompensation der detektierten Verschiebung.

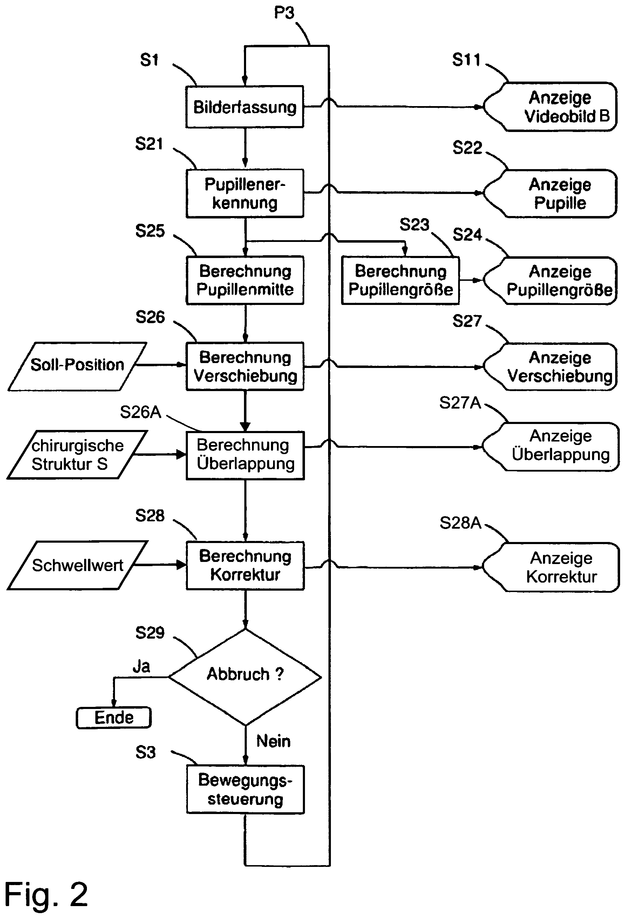

In **Fig. 2** ist der beispielhafte Ablauf eines Verfahrens zur Bewegungssteuerung in Form eines Flussdiagramms schematisch dargestellt. Die Schritte entsprechen weitgehend den in WO 2008/055604 A1 beschriebenen, wobei die Anzeigedaten in den Schritten S11, S22, S24 und S27 gleichzeitig angezeigt werden. Beispielsweise werden die erkannte Pupille und die ermittelte Verschiebung dem Videobild im identischen Maßstab überlagert und daneben die übrigen Daten eingeblendet. Zusätzlich werden die Schritte S26A, S27A und S28A durchgeführt, die nachfolgend erläutert werden. Dabei wird die zu erzeugende Struktur im Rahmen des Schrittes S27A zur Anzeige Überlappung ebenfalls dem Videobild überlagert. Wie in WO 2008/055604 A1 kann auch hier die Anzeige zusätzlich oder alternativ im Okular 6 erfolgen, wobei das Videobild im Okular 6 entfällt, da dort das optische Bild verfügbar ist.

In Schritt S26A wird anhand der erkannten Pupille als geometrischem Charakteristikum und der vorgegebenen chirurgischen Struktur S der momentane Grad der Überlappung zwischen der Struktur S und der momentanen optischen Zone des Auges A ermittelt und angezeigt, beispielsweise durch Schraffur der Schnittmenge der in das Videobild projizierten Flächen der Struktur S und der erkannten Pupille P. Der Grad der Überlappung kann in der Berechnung der Korrektur in Schritt S28 im Vergleich zu einem vorgegebenen Schwellwert verwendet werden. Der Schwellwert beschreibt beispielsweise eine mindestens zu erreichende Überlappung. Kann diese durch keine Bewegung erreicht werden, wird das Verfahren abgebrochen. Der ermittelte Korrekturvorschlag wird anderenfalls in Schritt S28A mit den anderen Daten angezeigt, beispielsweise als Verschiebungsvektor im Videobild.

Alternativ zu einer vollautomatischen Kompensationsbewegung kann auch ein Signal für den Anwender ausgegeben werden. Das Signal kann haptischer, optischer oder akustischer Art sein. Insbesondere ist eine quantitative Angabe über das Maß der gefundenen Abweichung vorgesehen, insbesondere eine gleichzeitige Videoüberlagerung von erkannter Pupille und erwarteter Behandlungsgeometrie, beispielsweise dem Lentikeldurchmesser, dem Flapdurchmesser, oder den Mittelpunkten.

Die Erfindung kann auch bei Excimer-Lasersystemen eingesetzt werden, bei denen die Formveränderung der Hornhaut durch Ablation der Cornea erfolgt. Zwar besteht dort nicht die Aufgabe, den Patienten bezüglich der Therapieoptik mit hoher Genauigkeit zu positionieren, da das Therapiegerät üblicherweise mittels Beaufschlagung der Strahlpositionierung mit einem durch Bewegungsverfolgung gemessenen Abweichungsbetrag kleinere Fehlpositionierungen ausgleicht, doch der Vergleich der Form und/oder Größe der detektierten Pupille hinsichtlich Form und Lage mit dem zur Ausführung vorgesehenen chirurgischen Struktur - beim Excimerlaser ein Ablationsmuster (engl. "ablation pattern") - kann auch bei solchen Behandlungsverfahren zur Verbesserung der Behandlungssicherheit und Behandlungswirksamkeit eingesetzt werden. Auch dann ist der Verzicht auf Beleuchtung im sichtbaren Spektralbereich und Anwendung infraroter Beleuchtung sinnvoll, um die bei Nachtsicht des Patienten wirksame optische Zone korrekt zu erfassen.

In einer weiteren Ausgestaltung werden auch andere Merkmale des Auges erkannt, beispielsweise Irisstrukturen. Diese werden dazu verwendet, die relative Drehlage des Auges mit gleichartiger Information aus Diagnosemessungen zu vergleichen, um auf diese Weise relative Verdrehung und relative Verschiebung bezüglich einer Diagnoseaufnahme (Registrierbild) zu bestimmen.

In einer weiteren Ausgestaltung wird diese Information dazu verwendet, um eine Drehung und/oder Verschiebung der Behandlungsgeometrie derart durchzuführen, dass die gefundene relative Verdrehung und/oder Verschiebung gegenüber Diagnosemessungen kompensiert wird.

In einer weiteren Ausgestaltung werden die erkannten Merkmale des Auges dazu verwendet, im Vergleich mit bekannten Merkmalen des geplanten Patientenauges die Identität des zu behandelnden Auges zu überprüfen (Augenerkennung). In diesem Fall erhält der Anwender eine Angabe über die Irrtumswahrscheinlichkeit oder das gefundene Maß der Identität.

**Fig. 3** zeigt zwei Varianten der Zentrierung eines Lentikels als zu erzeugender Struktur S, in Teilfigur 3A nahe dem Zentrum der photopischen Pupille P, in Teilfigur 3B nahe dem Zentrum der scotopischen Pupille P'. Man erkennt, dass im Fall A Lentikel und scotopische Pupille P' gerade noch überlappen. Die exakte Bewertung dieser oder ähnlicher Situationen gelingt dem Behandler nur dann in der ihm dafür zu Verfügung stehenden Zeit, wenn ihm die erfinderische Lösung zur Verfügung steht.

Zur Beschreibung der Erzeugung der Laserstrahlung wird auf WO 2008/055604 A1 verwiesen, dort insbesondere auf Fig. 6 und 7 und die zugehörigen Erläuterungen.

### Bezugszeichenliste

- 1: Laser-Behandlungseinrichtung
- 2: Lagerungseinrichtung
- 3: Behandlungslaser
- 4: Positioniereinrichtung
- 5: Behandlungskopf
- 6: Beobachtungsokular
- 7: Steuereinheit
- 8: Tastatur
- 9: Monitor
- 10: Kontaktelement
- 11: Detektionseinrichtung
- 12: Lichtquelle
- 13: Strahlteiler
- 14: Strahlteiler
- 15: Beobachtungsstrahlengang
- 19: Strahlteiler
- 20: Auswerteeinheit

- A: Auge
- B: Videobild
- P: Pupille
- S: Chirurgische Struktur

## Patentansprüche

1. Ophthalmologische Laser-Behandlungseinrichtung (1), aufweisend
- einen Behandlungslaser (3), insbesondere einen Excimerlaser oder einen Femtosekunden-Laser, zum Eintragen von Energie in einen Teil eines Auges (A) eines Patienten gemäß einer vorgegebenen chirurgischen Struktur (S),
- eine Lichtquelle (5) zum Beleuchten zumindest des Teils des Auges (A) und
- eine Detektionseinrichtung (6) zum Aufnehmen eines Bildes (B) zumindest des Teils des Auges (A),
**gekennzeichnet durch** eine Auswerteeinheit (20), die eingerichtet ist zum Ermitteln eines Grades einer momentanen Überlappung einer optischen Zone (P) des Auges (A) und der Struktur (S) oder zumindest eines refraktiv korrigierend wirkenden Teils der Struktur (S) anhand eines aufgenommenen Bildes (B).

2. Ophthalmologische Behandlungseinrichtung (1) nach Anspruch 1, wobei die Auswerteeinheit (20) zum Ermitteln des Grades der Überlappung eine Fläche der Pupille (P) anhand des aufgenommenen Bildes (B) und eine Fläche der Struktur (S) oder zumindest des refraktiv korrigierend wirkenden Teils der Struktur (S) und eine Schnittmenge der beiden Flächen ermittelt.

3. Ophthalmologische Behandlungseinrichtung (1) nach Anspruch 1 oder 2, wobei die Auswerteeinheit (20) eingerichtet ist zum Identifizieren und Lokalisieren eines Charakteristikums des Auges (A), insbesondere einer Pupille (P), in dem aufgenommenen Bild und zum Ermitteln einer relativen Verschiebung zwischen einem Punkt des Charakteristikums und einem Punkt der Struktur (S).

4. Ophthalmologische Behandlungseinrichtung (1) nach dem vorhergehenden Anspruch, wobei die Auswerteeinheit (20) das aufgenommene Bild (B) in Überlagerung mit einer grafischen Darstellung der Struktur (S) gemäß der ermittelten Verschiebung visuell ausgibt.

5. Ophthalmologische Behandlungseinrichtung (1) nach Anspruch 2 und/oder Anspruch 3, weiter umfassend eine Lagerungseinrichtung (2) für den Patienten und eine Positioniereinrichtung (4) zum Verschieben der Lagerungseinrichtung (2) und/oder des Lasers (3), wobei die Auswerteeinheit (20) die Positioniereinrichtung (4) in Abhängigkeit des ermittelten Grades der Überlappung von Pupille (P) und Struktur (S) und/oder der Verschiebung zwischen Charakteristikum und Struktur (S) steuert.

6. Ophthalmologische Behandlungseinrichtung (1) nach einem der vorhergehenden Ansprüche, wobei die Auswerteeinheit (20) einen Wert des ermittelten Grades mit einem vorgegebenen Schwellwert vergleicht und in Abhängigkeit eines Ergebnisses des Vergleichs ein haptisches und/oder visuelles und/oder akustisches Signal ausgibt und/oder wobei die Auswerteeinheit (20) einen Wert des ermittelten Grades mit einem vorgegebenen Schwellwert vergleicht und in Abhängigkeit eines Ergebnisses des Vergleichs, insbesondere auch in Abhängigkeit einer relativen Verschiebung, eine Bestrahlung des Auges (A) gemäß der Struktur (S) durchführt, insbesondere mit Identität der beiden Schwellwerte.

7. Ophthalmologische Behandlungseinrichtung (1) nach einem der vorhergehenden Ansprüche, umfassend ein Kontaktelement (10) zur mechanischen Fixierung des Auges (A).

8. Ophthalmologische Behandlungseinrichtung (1) nach dem vorhergehenden Anspruch, wobei die Auswerteeinheit (20) dazu eingerichtet ist, das Ermitteln des Grades der momentanen Überlappung vor und/oder nach dem mechanischen Fixieren durchzuführen.

9. Vorrichtung nach einem der vorhergehenden Ansprüche, wobei die chirurgische Struktur ein Symmetriezentrum aufweist, das frei ist von zu bestrahlenden Stellen.

## Claims

1. Ophthalmological laser treatment device (1), having
- a treatment laser (3), in particular an excimer laser or femtosecond laser, for introducing energy into a part of an eye (A) of a patient according to a predetermined surgical structure (S),
- a light source (5) for illuminating at least the part of the eye (A) and
- a detection device (6) for recording an image (B) of at least the part of the eye (A),
**characterized by** an evaluation unit (20) that is configured to ascertain a degree of a current overlap of an optical zone (P) of the eye (A) and the structure (S) or at least a part of the structure (S) with a refractively correcting effect on the basis of a recorded image (B).

2. Ophthalmological treatment device (1) according to Claim 1, wherein the evaluation unit (20) for ascertaining the degree of overlap ascertains an area of the pupil (P) on the basis of the recorded image (B) and an area of the structure (S) or at least of the part of the structure (S) with a refractively correcting effect and an intersection of the two areas.

3. Ophthalmological treatment device (1) according to Claim 1 or 2, wherein the evaluation unit (20) is configured to identify and localize a characteristic of the eye (A), in particular of a pupil (P), in the recorded image and to ascertain a relative displacement between a point of the characteristic and a point of the structure (S).

4. Ophthalmological treatment device (1) according to the preceding claim, wherein the evaluation unit (20) visually outputs the recorded image (B) in a superposition with a graphical illustration of the structure (S) according to the ascertained displacement.

5. Ophthalmological treatment device (1) according to Claim 2 and/or Claim 3, further comprising a bearing device (2) for the patient and a positioning device (4) for displacing the bearing device (2) and/or the laser (3), wherein the evaluation unit (20) controls the positioning unit (4) depending on the ascertained degree of overlap between the pupil (P) and structure (S) and/or the displacement between the characteristic and structure (S).

6. Ophthalmological treatment device (1) according to any one of the preceding claims, wherein the evaluation unit (20) compares a value of the ascertained degree to a predetermined threshold value and, depending on a result of the comparison, outputs a haptic and/or visual and/or acoustic signal and/or wherein the evaluation unit (20) compares a value of the ascertained degree to a predetermined threshold value and, depending on a result of the comparison, in particular also depending on a relative displacement, carries out an irradiation of the eye (A) according to the structure (S), in particular with the two threshold values being identical.

7. Ophthalmological treatment device (1) according to any one of the preceding claims, comprising a contact element (10) for mechanically fixing the eye (A).

8. Ophthalmological treatment device (1) according to the preceding claim, wherein the evaluation unit (20) is configured to carry out the ascertainment of the degree of the current overlap before and/or after the mechanical fixing.

9. Apparatus according to any one of the preceding claims, wherein the surgical structure has a centre of symmetry that is freed from places to be irradiated.

## Revendications

1. Appareil (1) de traitement ophtalmologique au laser comprenant :
- un laser thérapeutique (3), en particulier un laser à excimère ou un laser femtoseconde, visant à introduire de l'énergie dans une partie d'un oeil (A) d'un patient conformément à une structure chirurgicale (S) prédéfinie,
- une source de lumière (5) visant à illuminer au moins la partie de l'oeil (A) et
- un dispositif de détection (6) visant à recevoir une image (B) d'au moins la partie de l'oeil (A), **caractérisé par** une unité de traitement (20), laquelle est disposée afin de déterminer un degré d'un recouvrement momentané d'une zone optique (P) de l'oeil (A) et de la structure (S) ou d'au moins une partie de la structure (S) exerçant une correction de réfraction au moyen d'une image reçue (B).

2. Appareil (1) de traitement ophtalmologique selon la revendication 1, dans lequel l'unité de traitement (20) destinée à la détermination d'un degré d'un recouvrement détermine une surface de la pupille (P) au moyen de l'image reçue (B) et une surface de la structure (S) ou au moins de la partie de la structure (S) exerçant une correction de réfraction et un recoupement des deux surfaces.

3. Appareil (1) de traitement ophtalmologique selon la revendication 1 ou 2, dans lequel l'unité de traitement (20) est disposée afin d'identifier et de localiser un élément caractéristique de l'oeil (A), en particulier une pupille (P), dans l'image reçue et afin de déterminer un déplacement relatif entre un point de l'élément caractéristique et un point de la structure (S).

4. Appareil (1) de traitement ophtalmologique selon la revendication précédente, dans lequel l'unité de traitement (20) fournit visuellement l'image reçue (B) en surimpression avec une représentation graphique de la structure (S) selon le déplacement déterminé.

5. Appareil (1) de traitement ophtalmologique selon la revendication 2 et/ou la revendication 3, comprenant en outre un dispositif de placement (2) pour le patient et un dispositif de positionnement (4) afin de déplacer le dispositif de placement (2) et/ou le laser (3), dans lequel l'unité de traitement (20) commande le dispositif de positionnement (4) en fonction du degré de recouvrement déterminé entre la pupille (P) et la structure (S) et/ou le déplacement entre l'élément caractéristique et la structure (S).

6. Appareil (1) de traitement ophtalmologique selon l'une des revendications précédentes, dans lequel l'unité de traitement (20) compare une valeur du degré déterminé avec une valeur seuil prédéfinie et émet un signal haptique et/ou visuel et/ou acoustique en fonction d'un résultat de la comparaison et/ou dans lequel l'unité de traitement (20) compare une valeur du degré déterminé avec une valeur seuil prédéfinie et effectue, en fonction d'un résultat de la comparaison, en particulier également en fonction d'un déplacement relatif, une irradiation de l'oeil (A) conformément à la structure (S), en particulier avec les deux valeurs de seuil identiques.

7. Appareil (1) de traitement ophtalmologique selon l'une des revendications précédentes, comprenant un élément de contact (10) visant à fixer mécaniquement l'oeil (A).

8. Appareil (1) de traitement ophtalmologique selon l'une des revendications précédentes, dans lequel l'unité de traitement (20) est disposée de façon à effectuer la détermination du degré d'un recouvrement momentané avant et/ou après la fixation mécanique.

9. Dispositif selon l'une des revendications précédentes, dans lequel la structure chirurgicale comporte un centre de symétrie, lequel est libre de positions à irradier.
